(19) Europäisches Patentamt
European Patent Office
Office européen des brevets

(11) Publication number: **0 029 362**
**B1**

(12) # EUROPEAN PATENT SPECIFICATION

(45) Date of publication of patent specification: **07.09.83**

(51) Int. Cl.$^3$: **C 07 C 69/83**, C 07 C 69/767, C 07 C 67/03, B 01 J 31/26

(21) Application number: **80304105.2**

(22) Date of filing: **14.11.80**

(54) **Production of a diallyl ester of an aromatic dicarboxylic acid.**

(30) Priority: **15.11.79 JP 147026/79**

(43) Date of publication of application:
**27.05.81 Bulletin 81/21**

(45) Publication of the grant of the patent:
**07.09.83 Bulletin 83/36**

(84) Designated Contracting States:
**DE FR GB IT SE**

(56) References cited:
**DE - B - 1 211 625**
**FR - A - 1 520 215**

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Seguchi, Koji**
**687-19 Miyawada Fujishiro-machi**
**Kita-Sohma-gun, Ibaraki-ken (JP)**

(74) Representative: **Myerscough, Philip Boyd et al,**
**J.A.Kemp & Co. 14, South Square Gray's Inn**
**London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 029 362 B1**

Courier Press, Leamington Spa, England

Production of a diallyl ester of an aromatic dicarboxylic acid

The present invention relates to a process for producing a diallyl ester of an aromatic dicarboxylic acid by heating a dialkyl ester of an aromatic dicarboxylic acid with an allyl alcohol.

Polymerization products of diallyl esters of a symmetrical aromatic dicarboxylic acid, for instance, of diallyl terephthalic acid, possess excellent electrical properties, dimensional stability, heat resistance, weather-proofness, resistance to chemicals and mechanical properties compared with the polymerization products of diallyl esters of a non-symmetrical aromatic dicarboxylic acid. Accordingly, the former have been widely utilized as electrical, mechanical and constructional materials. Further it has recently been found that polymerization products of diallyl esters of a symmetrical aromatic dicarboxylic acid also possess excellent optical properties such as transparency, refractive index and surface hardness. Use of the polymer as an optical material is expected.

Conventional methods for producing diallyl esters of aromatic dicarboxylic acids include:

(1) direct esterification of an aromatic dicarboxylic acid with an allyl alcohol,

(2) condensation of a metal salt of an aromatic dicarboxylic acid with an allyl halide in the presence of a tertiary amine catalyst in an aqueous medium or in an anhydrous system, and

(3) ester-exchange between an ester of an aromatic dicarboxylic acid and an allyl alcohol.

The method of direct esterification (1) proceeds slowly. It is necessary to carry out the reaction at a high temperature for a long period of time causing unfavorable phenomena such as polymerization of the allyl alcohol and colouring of the reaction product. Moreover, the reactivity of symmetrical aromatic dicarboxylic acid and allyl alcohol in this direct reaction is poor.

FR—A—152015 discloses a process for the production of a polyallyl ester of a benzenepolycarboxylic acid by reacting an excess of allyl alcohol with a benzenepolycarboxylic acid or anhydride at a temperature of 165 to 250°C in the absence of a strongly acidic catalyst having a $pK_a$ below 4, the reaction being carried out in the presence of an esterification catalyst having a $pK_a$ above 4 when temperatures below 190°C are employed, for a time sufficient to convert at least 90% of the acid to the polyallyl ester.

The condensation method (2) for producing diallyl esters of aromatic dicarboxylic acids has been and is now the method most generally adopted. However, the method has its demerits. Reaction in an anhydrous system necessitates troublesome processes such as condensing and drying the metal salt of an aromatic dicarboxylic acid prepared in an aqueous solution. Where the reaction is carried out in an aqueous medium, by-products derived from hydrolysis of the allyl halide tend to form and, accordingly, there are problems of separation of by-products and purification of the main product. Moreover, the metal salt of symmetrical aromatic dicarboxylic acid used as a starting material is hard to synthesize. The method has not yet been adopted industrially.

As far as the ester-exchange method (3) is concerned its starting materials are easily available and it can be carried out under mild reaction conditions. However, the use of a catalyst is essential and it is no exaggeration to say that industrial values such as productivity, cost, etc. are mainly determined by the selection of the catalyst. Hitherto, sodium methoxide, metallic magnesium, tetrabutyl titanate and organic tin compounds have been proposed as catalysts. According to the results of tracing experiments effected by the present inventor, these known catalysts have various disadvantages in the ester-exchange reaction (with an ester of symmetrical aromatic dicarboxylic acid and allyl alcohol) such as their lower catalytic activity so not to give a high yield and their tendency to cause formation of by-products and polymers and colouration.

DE—B—1211625 discloses a process for the preparation of diallyl esters of carbocyclic aromatic dicarboxylic acids by catalytically transesterifying a dimethyl and/or diethyl ester of the acid with at least an equivalent amount of allyl alcohol, calculated with reference to the methyl or ethyl ester groups, in an inert atmosphere by a gradual addition of allyl alcohol at a temperature above its boiling point up to 170°C, with the evolution and removal of the methanol or ethanol, so that a diallyl ester is formed. The catalyst is magnesium alkoxide, metallic magnesium or butyl titanate present in an amount of 0.01 to 5.0%, preferably 0.1 to 1%, by weight based on the weight of the ester.

It has now been found that these problems can be overcome by producing a diallyl ester of an aromatic dicarboxylic acid, particularly of a symmetrical aromatic dicarboxylic acid, by heating with an allyl alcohol a dialkyl ester of an aromatic dicarboxylic acid of the formula:

$$R^3OOC{-}(Ar){-}COOR^4$$

wherein Ar is phenylene, naphthylene, biphenylene, anthrylene, phenanthrylene or acenaphthenylene and $R^3$ and $R^4$ represent the same or a different alkyl group of from 1 to 3 carbon atoms in the presence of, as catalysts, both (A) at least one organic tin compound of the formula (I)

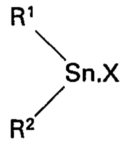

(I)

wherein $R^1$ and $R^2$ independently represent alkyl of 1 to 8 carbon atoms or phenyl, and X represents two halogen atoms or an oxygen atom, and (B) at least on substance which is an element or an oxide, alkoxide or acetate of magnesium, zinc, tin, lead, aluminum, nickel or zirconium.

The use of Catalysts (A) and (B) accelerates the ester-exchange reaction while suppressing side-reactions such as polymerization and addition, thus giving the intended product at a high yield.

In the description which follows, reference will be made to the accompanying drawings, in which:

Figure 1 shows the composition of the reaction mixture with the passage of time in Example 1 (Curves A—1, A—2 and A—3) and Comparative Example 3 (Curves B—1, B—2 and B—3), wherein A—1 shows the relationship between the amount of starting material and the time of reaction, A—2 shows the relationship between the amount of diallyl terephthalate and the time of reaction, A—3 shows the relationship between the amount of allyl methyl terephthalate and the time of reaction in Example 1, and B—1, B—2 and B—3 respectively show the same relationship in Comparative Example 3 as A—1, A—2 and A—3 in Example 1;

Figure 2 shows in infrared absorption spectrogram of the diallyl terephthalate obtained in Example 1;

Figure 3 shows the composition of the reaction mixture with the passage of time in Example 3 (C—1, C—2 and C—3) and in Comparative Example 4 (D—1, D—2 and D—3), wherein C—1, C—2 and C—3 respectively show the same relationship in Example 3 and D—1, D—2 and D—3 respectively show the same relationship in Comparative Example 4 as A—1, A—2 and A—3 in Example 1 and

Figure 4 shows an infrared absorption spectrogram of diallyl 2,6-napthalenedicarboxylate obtained in Example 5.

The ester of an aromatic dicarboxylic acid for use in the present invention is a dialkyl ester represented by the general formula (II):

$$R^3OOC\text{-}(Ar)\text{-}COOR^4 \qquad (II)$$

wherein $R^3$ and $R^4$ represents the same or different alkyl group of 1 to 3 carbon atoms, and Ar represents a group selected from phenylene, naphthylene, biphenylene, anthrylene, phenanthrylene and acenaphthenylene. The acid component of such esters includes phthalic-, isophthalic-, terephthalic-, 2,6-naphthlenedicarboxylic-, 2,7-naphthalenedicarboxylic-, 1,5-naphthalenedicarboxylic acid, biphenyldicarboxylic acids, anthracenedicarboxylic acids and acenaphthenedicarboxylic acids. On the other hand, the alcohol component of such esters includes methanol, ethanol, propanol and isopropyl alcohol.

The ester of an aromatic dicarboxylic acid is used singly or as a mixture of two or more such esters. In addition, the acidic component of esters of a symmetrical aromatic dicarboxylic acid includes terephthalic-, 2,6-naphthalenedicarboxylic-, 4,4′-biphenyldicarboxylic-, and 9,10-, 1,5- or 2,6-anthracenedicarboxylic acid.

The allyl alcohol is preferably allyl alcohol itself $CH_2=CH—CH_2OH$. However, it has been found that also where methallyl alcohol is used instead of allyl alcohol in an ester-exchange reaction in the presence of Catalyst (A) and Catalyst (B), di-methallyl ester of an aromatic dicarboxylic acid can be readily obtained at a high yield in a relatively short time of reaction.

The organic tin compound represented by the general formula (I) for use as Catalyst (A) is, for instance, dimethyltin dichloride, diethyltin dichloride, bibutyltin dichloride, dimethyltin dibromide, dibutyltin dibromide, dioctyltin dichloride, diphenyltin dichloride, dimethyltin oxide, diethyltin oxide, dibutyltin oxide, dioctyltin oxide or diphenyltin oxide. A single compound or a mixture of two or more of these compounds can be used in the present invention.

The Catalyst (B) for use in the present invention together with Catalyst (A) is an element or an oxide, alkoxide or acetate of magnesium, zinc, tin, lead, aluminum, nickel or zirconium. More preferably, the catalyst is a simple substance such as metallic magnesium, magnesium methoxide, zinc duct, zinc oxide, metallic tin, tin oxide, lead acetate, aluminum oxide, nickel oxide aluminum ethoxide or zirconium acetate. A mixture of such elements and/or compounds can be used.

The production of a diallyl ester of an aromatic dicarboxylic acid according to the present invention can be very simply carried out. For example more than two moles of an allyl alcohol and one mole of the ester of an aromatic dicarboxylic acid are mixed and the mixture is heated in the presence of both Catalyst (A) and Catalyst (B) at a temperature of 100 to 200°C, preferably of 110 to 150°C, for 1 to 25 hours, preferably for 3 to 20 hours, to cause reaction. The molar ratio of the allyl alcohol to the ester is theoretically 2:1. However, in order to accelerate the reaction and to use the allyl alcohol also as a solvent, the actual molar ratio is preferably 2.5:1 to 15:1.

The amount of Catalyst (A) used in the reaction is preferably 0.3 to 5 mole%, preferably 0.5 to 3 mole%, of the ester of aromatic dicarboxylic acid because of its smaller effectiveness at concentrations of less than 0.3 mole%, and of no more improvement in

effectiveness at concentrations of more than 5 mole%.

The amount of Catalyst (B) used in the reaction is preferably 0.01 to 5 mole% of Catalyst (A), preferably 0.1 to 2 mole%. The reason for selecting these ranges is the same as for Catalyst (A). However, where Catalyst B does not dissolve in the allyl alcohol in the reaction system, the amount should be a little more, say 0.5 to 2 mole%.

The reaction is usually carried out under a normal pressure and reflux-condensing of the allyl alcohol. However, in order to improve the reaction efficiency, the system can be vigorously agitated or the alcohol that is formed can be distilled out of the system as soon as it is formed and additional charge of allyl alcohol is added continuously, at least one rectifying column being provided to the reactor as a means for distilling off the alcohol.

After the reaction is over, the products are separate from the reaction mixture by distillation or recrystallization. In the case where the main product is liquid, it can be purified by distillation under reduced pressure from the reaction mixture. On the other hand, the case where the main product separates as crystals, as in the case of diallyl 2,6-naphthalate, recrystallization can be carried out to isolate and purify the product while using an organic solvent.

As has been stated, a diallyl ester of an aromatic dicarboxylic acid can be obtained according to the present invention within a very short period of time at a high yield, resulting in a useful contribution to industry.

The following Examples illustrate the present invention:

Example 1

Production of diallyl terephthalate

$7.76 \times 10^{-2}$Kg (77.6 g) of dimethyl terephthalate, $9.28 \times 10^{-2}$ kg (92.8 g) of allyl alcohol, $1.21 \times 10^{-3}$ kg (1.21 g) of dibutyltin dichloride and $1.3 \times 10^{-4}$ kg (0.13 g) of zinc dust were introduced into a three-necked flask provided with rectifying columns on two of the necks. The contents of the flask were heated at 130°C using an oil bath. The distillation of methanol from the flask through the rectifying column continued for 5 hours, amounting to $3.19 \times 10^{-5} m^3$ (31.9 ml) of distilled methanol. Gas-chromatographical data showed that the reaction mixture at the end of the distillation of methanol consisted of 99.5% by weight of the desired product, diallyl terephthalate, and of 0.5% by weight of a by-product, allyl methyl terephthalate. No other impurities were detected by gas chromatography.

During the above-mentioned reaction, sampling of the reaction mixture was carried out every hour from the starting of heating. The sample specimens were directly subjected to examination by gas-chromatography to determine the respective amounts of unreacted ester, produced diallyl terephthalate and by-product allyl methyl terephthalate versus the time of heating. The results of the determination are shown in Figure 1 by Curves A with the conditions of gas-chromatography being a rate of temperature rise of 1/6°C/sec (10°C/min) and a temperature range of 100 to 250°C. A Model SE—30 gas-chromatographic apparatus made by Gasukuro Industry Industry Co. Japan was used.

After the reaction was over, excess allyl alcohol in the flask was removed by heating under reduced pressure. The remaining crude product in the flask was separated from zinc dust by filtration. The filtrate was directly distilled under reduced pressure of $6.666 \times 10^2$Pa (5 mmHg) while collecting the fraction distilling at a boiling point of 162 to 164°C. The fraction amounted to $9.61 \times 10^{-2}$ kg (96.1 g).

The thus obtained product was clear and colourless liquid of specific gravity of 1.119, of refractive index of $n_D^{20°}$ of 1.5283, of a viscosity at 24°C of $1.5 \times 10^{-2}$ Pa s (15 cP). The elementary analytical data, the gas-chromatographic analytical data and infrared absorption spectrum of the compound coincided with those of an authenticated specimen of diallyl terephthalate. Figure 2 shows an infrared absorption spectrum of diallyl terephthalate obtained in Example 1 according to the present invention.

Comparative Example 1

Production of diallyl terephthalate

A mixture $7.76 \times 10^2$ kg (77.6 g) of dimethyl terephthalate, $9.28 \times 10^{-2}$ kg (92.8 g) of allyl alcohol and $1.51 \times 10^{-3}$ kg (1.51 g) of dibutyltin dichloride was heated as in Example 1. After heating for 10 hours, the reaction mixture was subjected directly to gas-chromatography to find that the reaction mixture was composed of 54.1% by weight of diallyl terephthalate, 19.3% by weight of allyl methyl terephthalate and 26.6% by weight of unreacted substances.

Comparative Example 2

Production of diallyl terephthalate

A mixture of $7.76 \times 10^{-2}$ (77.6 g) of dimethyl terephthalate $9.28 \times 10^{-2}$ kg (92.8 g) of allyl alcohol and $2.6 \times 10^{-4}$ kg (0.26 g) of zinc dust was heated as in Example 1 for 10 hours. Then, the reaction mixture was directly subjected to gas-chromatography to find that the reaction mixture was composed of 15.1% by weight of diallyl terephthalate, 53.5% by weight of allyl methyl terephthalate and the balance being unreacted substances.

Comparative Example 3

Production of diallyl terephthalate

A mixture of $7.96 \times 10^{-2}$ kg (79.6 g) of dimethyl terephthalate, $9.28 \times 10^{-2}$ kg (92.8 g) of allyl alcohol, $1.21 \times 10^{-3}$ kg (1.21 g) of dibutyltin dichloride and $2.2 \times 10^{-4}$ kg (0.22 g) of sodium methylate was heated for 9 hours as in Example 1. The reaction mixture was then

examined directly by gas-chromatography to find that the reaction mixture was composed of 95.3% by weight of diallyl terephthalate, 4.5% by weight of allyl methyl terephthalate and the balance of unreacted substances. The amount of methanol recovered was $3.13 \times 10^{-5}$ $m^3$ (31.3 ml).

As in Example 1, sampling of the reaction mixture and gas-chromatographic examination of the samples were carried out every hour from the commencement of heating, and the results of the examination are shown in Figure 1 by Curve B.

As are clearly seen in Example 1 and Comparative Examples 1 to 3, it was necessary to carry out the reaction for a longer period of time in the cases where the organic tin compound or metal was used singly as in Comparative Examples 1 and 2, than in the case where the reaction was carried out in the presence of both Catalysts (A) and (B) as in Example 1. The rate of reaction was slower where a metal other than those of the present invention was used.

Example 2

A mixture of $8.88 \times 10^{-2}$ kg (88.8 g) of diethyl terephthalate, $9.86 \times 10^{-2}$ kg (98.6 g) of allyl alcohol, $1.21 \times 10^{-3}$ kg (1.21 g) of dibutyltin dichloride and $3.2 \times 10^{-4}$ kg (0.32 g) of zinc oxide was heated as in Example 1 at 130°C for 5 hours. The reaction mixture was directly subjected to gas-chromatography to find that the reaction mixture was composed of 98.1% by weight of diallyl terephthalate and 1.9% of allyl ethyl terephthalate.

After filtering the reaction mixture to remove zinc oxide and partly undissolved dibutyltin dichloride, the filtrate was directly distilled under reduce pressure of $6.666 \times 10^2$ Pa (5 mmHg). The fraction distilling at 162 to 164°C at that pressure was $9.51 \times 10^{-2}$ kg (95.1 g). The infrared absorption spectrum, the elementary analytical data, the specific gravity and the refractive index of the thus obtained product coincided with those of an authenticated sample of diallyl terephthalate.

Example 3

A mixture of $7.76 \times 10^{-2}$ kg (77.6 g) of dimethyl terephthalate, $9.86 \times 10^{-2}$ kg (98.6 g) of allyl alcohol, $9.9 \times 10^{-4}$ kg (0.99 g) of dibutyltin oxide and $4.6 \times 10^{-4}$ kg (0.46 g) of tin oxide was heated as in Example 1 at 130°C for 5 hours. Sampling was carried out every hour from the commencement of heating. The samples were subjected directly to gas-chromatography to obtain information on the reaction shown by Curve C in Figure 3. After 5 hours of heating, the reaction mixture was distilled as in Example 1 to collect $9.52 \times 10^{-2}$ kg (95.2 g) of a colourless and transparent liquid at 162 to 164°C/$6.666 \times 10^2$ Pa (5 mmHg). The product was identified as diallyl terephthalate from the coincidence of its infrared spectrum, its elementary analytical data and its other properties with those of an authenticated sample of diallyl terephthalate.

Comparative Example 4

A mixture of $7.76 \times 10^{-2}$ kg (77.6 g) of dimethyl terephthalate, $9.86 \times 10^{-2}$ kg (98.6 g) of allyl alcohol, $1.23 \times 10^{-3}$ kg (1.23 g) of dibutyltin oxide was heated as in Example 3 at 130°C. During the heating, sampling of the reaction mixture was carried out every hour. The samples were directly subjected to gas-chromatography to obtain information on the composition of the reaction mixture with the passage of time. The information is shown in Figure 3 by Curves D. As is seen in Figure 3, in the present case using only one kind of catalyst, that is Catalyst (A), the rate of reaction was smaller than that in Example 3, and it took longer to complete the reaction than in Example 3.

Example 4

A mixture of $7.76 \times 10^{-2}$ kg (77.6 g) of dimethyl terephthalate, $6.96 \times 10^{-2}$ kg (69.6 g) of allyl alcohol, $9.2 \times 10^{-4}$ kg (0.92 g) of dibutyltin dichloride, $9.99 \times 10^{-4}$ kg (0.99 g) of dibutyltin oxide and $9.6 \times 10^{-5}$ kg (0.096 g) of metallic magnesium was heated as in Example 1 at 130°C for 5 hours. The distillation of methanol stopped after 5 hours of heating with the amount yielded being $3.21 \times 10^{-5}$ $m^3$ (32.1 ml). The gas-chromatographic information on the reaction mixture was 99.7% by weight of diallyl terephthalate and 0.3% by weight of allyl methyl terephthalate, impurities not being observed.

On distilling the reaction mixture directly under reduced pressure of $6.666 \times 10^2$ Pa (5 mmHg), the fraction distilling at 162 to 164°C was collected in an amount of $9.65 \times 10^{-2}$ kg (96.5 g). The thus obtained colourless and transparent liquid showed an infrared absorption spectrum, elementary analytical data and other properties coinciding with those of an authenticated sample of diallyl terephthalate.

Example 5

Production of diallyl 2,6-naphthalenedicarboxylate

A mixture of $2.42 \times 10^{-2}$ kg (24.2 g) of dimethyl 2,6-naphthalenedicarboxylate, $5.8 \times 10^{-2}$ kg (58.0 g) of allyl alcohol, $9.1 \times 10^{-4}$ kg (0.91 g) of dibutyltin dichloride, and $2.6 \times 10^{-4}$ kg (0.26 g) of magnesium methoxide was, in the same manner as in Example 1, heated at 130°C. After heating for 15 hours, distillation of methanol was over, the amount of distilled methanol being $6.7 \times 10^{-6}$ $m^3$ (6.7 ml). The result of direct chromatography of the reaction product showed the composition of 99.5% by weight of diallyl 2,6-naphthalenedicarboxylate and 0.5% by weight of allyl methyl 2,6-naphthalenedicarboxylate, other substances not being detected.

After removing excess allyl alcohol by distillation under reduced pressure, the crude product was dissolved in methanol while heating. After removing insoluble substances by filtration, the filtrate was cooled to obtain white needle-like crystals in an amount of $2.35 \times 10^{-2}$ kg (23.5 g). By comparing the data of infrared absorption spectrum, of elementary analysis and of nuclear magnetic resonance spectrum with those of an authenticated sample, the product was identified as diallyl 2,6-naphthalenedicarboxylate. Infrared absorption spectrum of the product is shown in Figure 4.

Elementary analysis, found: 73.0% of C and 5.4% of H
Calculated as $C_{18}H_{16}O_4$: 72.9% of C and 5.4% of H

Examples 6 to 9

Four runs for the production of diallyl terephthalate were respectively carried out by heating $3.88 \times 10^{-2}$ kg (38.8 g) of dimethyl terephthalate, and $4.64 \times 10^{-2}$ kg (46.4 g) of allyl alcohol with each of the organic tin compounds and each of the metals or metal compounds shown in Table 1 in the amounts also shown in Table 1 in the same manner as in Example 1. When the reaction was over, the reaction mixture was subjected to distillation under reduced pressure as in Example 1. The fraction distilling at 162 to 164°C/$6.666 \times 10^2$ Pa (5 mmHg) was collected to be examined by infrared absorption spectroscopy and elementary analysis. All the products in the four runs were identified as diallyl terephthalate.

The amount of diallyl terephthalate in the reaction mixture and the yield of distilled diallyl terephthalate of each run is shown also in Table 1.

TABLE 1

Conditions of synthesis of diallyl terephthalate and Results of Synthesis

| Example | Organic tin compound | | Metal or its compound | | Reaction time (hour) | Amount of ester[2] (kg) | Yield of the ester (%) |
|---|---|---|---|---|---|---|---|
| | Name | amount (kg) | Name | amount (kg) | | | |
| 6 | $(Octyl)_2SnCl_2$ | $2.48 \times 10^{-3}$ | Magnesium | $4.8 \times 10^{-5}$ | 5.0 | $46.3 \times 10^{-3}$ | 99.5 |
| 7 | $(Methyl)_2SnO$ | $6.5 \times 10^{-4}$ | $Al_2O_3$ | $2.0 \times 10^{-4}$ | 5.0 | $44.5 \times 10^{-3}$ | 98.6 |
| 8 | $(Phenyl)_2SnO$ | $8.4 \times 10^{-4}$ | $(CH_3COO)_2Pb$ | $4.4 \times 10^{-4}$ | 5.5 | $46.0 \times 10^{-3}$ | 99.1 |
| 9 | $(Octyl)_2SnO$ | $1.44 \times 10^{-3}$ | NiO | $1.4 \times 10^{-4}$ | 6.0 | $43.6 \times 10^{-3}$ | 96.5 |

Notes:

1) Yield of diallyl terephthalate in the reaction mixture, represented % by weight.

2) Amount of pure diallyl terephthalate distilling at 162 to 164°C/$6.666 \times 10^2$Pa.

**Claims**

1. A process for producing a diallyl ester of an aromatic dicarboxylic acid by heating with an allyl alcohol a dialkyl ester of an aromatic dicarboxylic acid of the formula:

$$R^3OOC\text{-}(Ar)\text{-}COOR^4$$

wherein Ar is phenylene, naphthylene, biphenylene, anthrylene, phenanthrylene or acenaphthenylene, and $R^3$ and $R^4$ represent the same or a different alkyl group of 1 to 3 carbon atoms; characterised in that reaction is effected in the presence of, as catalysts, both (A) at least one organic tin compound of the formula (I)

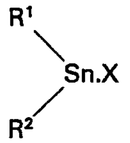
(I)

wherein $R^1$ and $R^2$ independently represent alkyl of 1 to 8 carbon atoms or phenyl, and X represents two halogen atoms or an oxygen atom, and (B) at least one substance which is an element or an oxide, alkoxide or acetate of magnesium, zinc, tin, lead, aluminum, nickel or zirconium.

2. A process according to claim 1, characterised in that the ester of an aromatic dicarboxylic acid is a symmetrical compound.

3. A process according to claim 1 or 2, characterised in that the organic tin compound of formula (I) is selected from dimethyltin dichloride, diethyltin dichloride, dibutyltin dichloride, dimethyltin dibromide, dibutyltin dibromide, dioctyltin dichloride, diphenyltin dichloride, dimethyltin oxide, diethyltin oxide, dibutyltin oxide, dioctyltin oxide and diphenyltin oxide.

4. A process according to any one of the preceding claims characterised in that the catalyst (B) is metallic magnesium, zinc dust, metallic tin, magnesium methoxide, zinc oxide, tin oxide, lead acetate, aluminum oxide, nickel oxide, aluminum ethoxide or zirconium acetate.

5. A process according to any one of the preceding claims characterised in that heating is effected at a temperature of 100 to 200°C.

**Patentansprüche**

1. Verfahren zur Herstellung eines Diallylesters einer aromatischen Dicarbonsäure durch Erhitzen eines Dialkylesters einer aromatischen Dicarbonsäure mit der Formel:

$$R^3OOC\text{-}(Ar)\text{-}COOR^4$$

in der Ar Phenylen, Naphthylen, Biphenylen, Anthrylen, Phenanthrylen oder Acenaphthenylen und $R^3$ und $R^4$ die gleichen oder verschiedene Alkylgruppen mit 1 bis 3 Kohlenstoffatomen bedeuten, mit einem Allylalkohol, dadurch gekennzeichnet, daß die Reaktion in Gegenwart sowohl von (A), mindestens einer organischen Zinnverbindung mit der Formel (I)

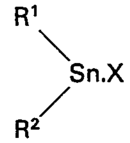
(I)

in der $R^1$ und $R^2$ unabhängig voneinander eine Alkylgruppe mit 1 bis 8 Kohlenstoffatomen oder eine Phenylgruppe und X zwei Halogenatome oder ein Saurestoffatom bedeuten, als auch von (B) mindestens einer Substanz, die ein Element oder ein Oxid, Alkoxid oder Acetat von Magnesium, Zink, Zinn, Blei, Aluminium, Nickel oder Zirkonium ist, als Katalysatoren durchgeführt wird.

2. Verfahren nach Anspruch 1, dadurch gekennzeichnet, daß der Ester einer aromatischen Dicarbonsäure eine symmetrische Verbindung ist.

3. Verfahren nach Anspruch 1 oder 2, dadurch gekennzeichnet, daß die organische Zinnverbindung mit der Formel (I) ausgewählt ist aus Dimethylzinndichlorid, Diethylzinndichlorid, Dibutylzinndichlorid, Dimethylzinndibromid, Dibutylzinndibromid, Dioctylzinndichlorid, Diphenylzinndichlorid, Dimethylzinnoxid, Diethylzinnoxid, Dibutylzinnoxid, Dioctylzinnoxid und Diphenylzinnoxid.

4. Verfahren nach jedem der vorangegangenen Ansprüche, dadurch gekennzeichnet, daß der Katalysator (B) metallisches Magnesium, Zinkstaub, metallisches Zinn, Magnesiummethoxid, Zinkoxid, Zinnoxid, Bleiacetat, Aluminiumoxid, Nickeloxid, Aluminiumethoxid oder Zirkoniumacetat ist.

5. Verfahren nach jeden der vorangegangenen Ansprüche, dadurch gekennzeichnet, daß das Erhitzen auf eine Temperatur von 100 bis 200°C erfolgt.

**Revendications**

1. Procédé de préparation d'un ester diallylique d'un acide aromatique dicarboxylique par chauffage avec un alcool allylique d'un dialkyl ester d'un acide dicarboxylique aromatique de formule:

$$R^3OOC\text{-}(Ar)\text{-}COOR^4$$

dans laquelle:

Ar représente un phénylène, un naphtylène, biphénylène, anthrylène, phénanthrylène ou acénaphténylène, et,

$R^3$ et $R^4$ représentent des groupements alkyle égaux ou différents possédant de 1 à 3 atomes de carbone,

caractérisé en ce que la réaction est effectuée en présence comme catalyseur à la fois de (A) au moins un composé organique de l'étain de formule (I):

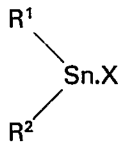

(I)

dans laquelle:

$R^1$ et $R^2$ représentent indépendamment un alkyle possédant de 1 à 8 atommes de carbone ou un phènyle, et,

X représente deux atomes d'halogène ou un atome d'oxygène, et (B) au moins une substance qui est un élément ou un oxyde, un alkoxyde ou un acétate de magnésium, zinc, étain, plomb, aluminium, nickel ou zirconium.

2. Procédé suivant la revendication 1, caractérisé en ce que l'ester d'un acide aromatique dicarboxylique est un composé symétrique.

3. Procédé suivant la revendication 1 ou 2, caractérisé en ce que le composé organique de l'étain de formule (I) est choisi parmi les, dichlorure d'étain, dichlorure de diéthylétain, dichlorure de dibutylétain, dibromure de diméthylétain, dibromure de dibutylétain, dichlorure de dioctylétain, dichlorure de diphénylétain, oxyde de diméthylétain, oxyde de diéthylétain, oxyde de dibutylétain, oxyde de dioctylétain, et oxyde de diphénylétain.

4. Procédé suivant l'une quelconque des revendications précédentes, caractérisé en ce que le catalyseur (B) est le magnésium métallique, la poudre de zinc, l'étain métallique, le méthoxyde de magnésium, l'oxyde de zinc, l'oxyde d'étain, l'acétate de plomb, l'oxyde d'aluminium, l'oxyde de nickel, l'éthoxyde d'aluminium ou l'acétate de zirconium.

5. Procédé suivant l'une quelconque des revendications précédentes, caractérisé en ce que le chauffage est effectué à une température de 100 à 200°C.

Fig. 1

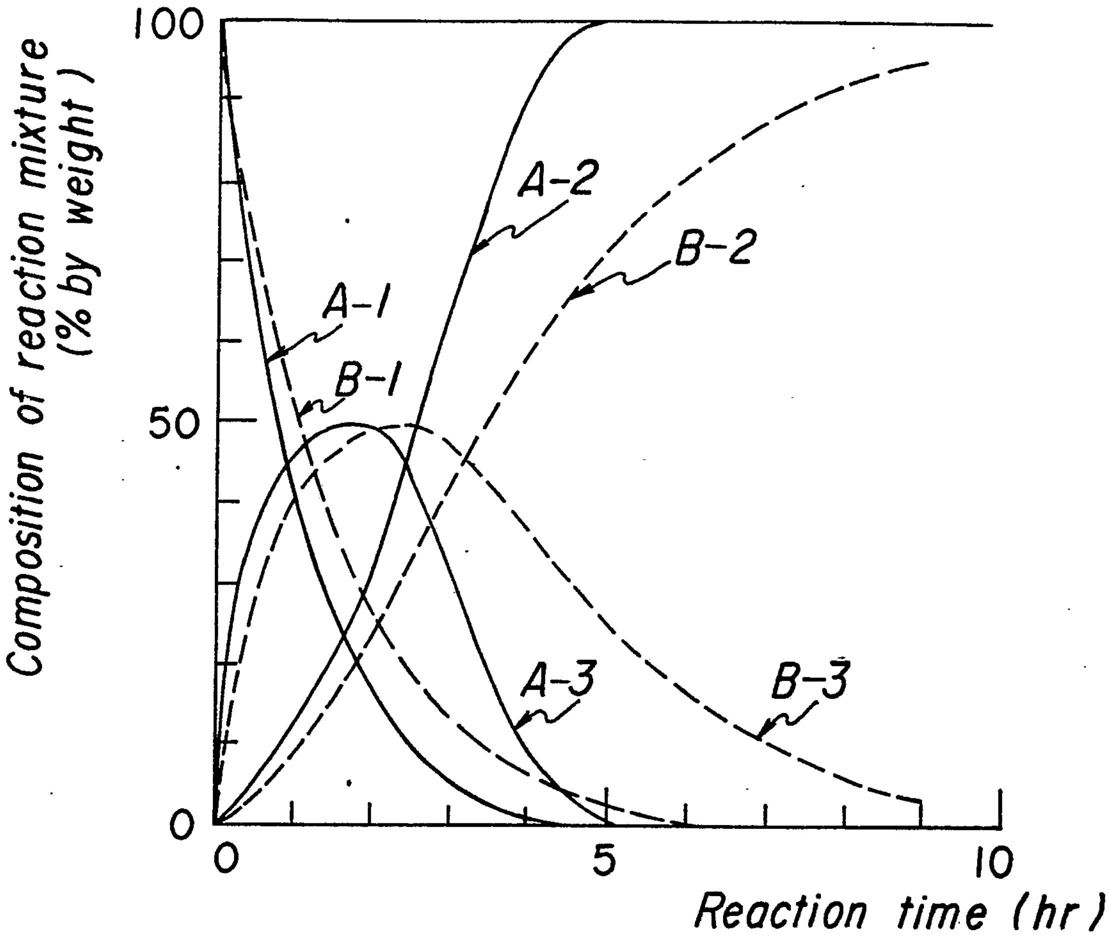
100
50
0
0
5
10
Composition of reaction mixture (% by weight)
Reaction time (hr)
A-1
A-2
A-3
B-1
B-2
B-3

Fig. 2

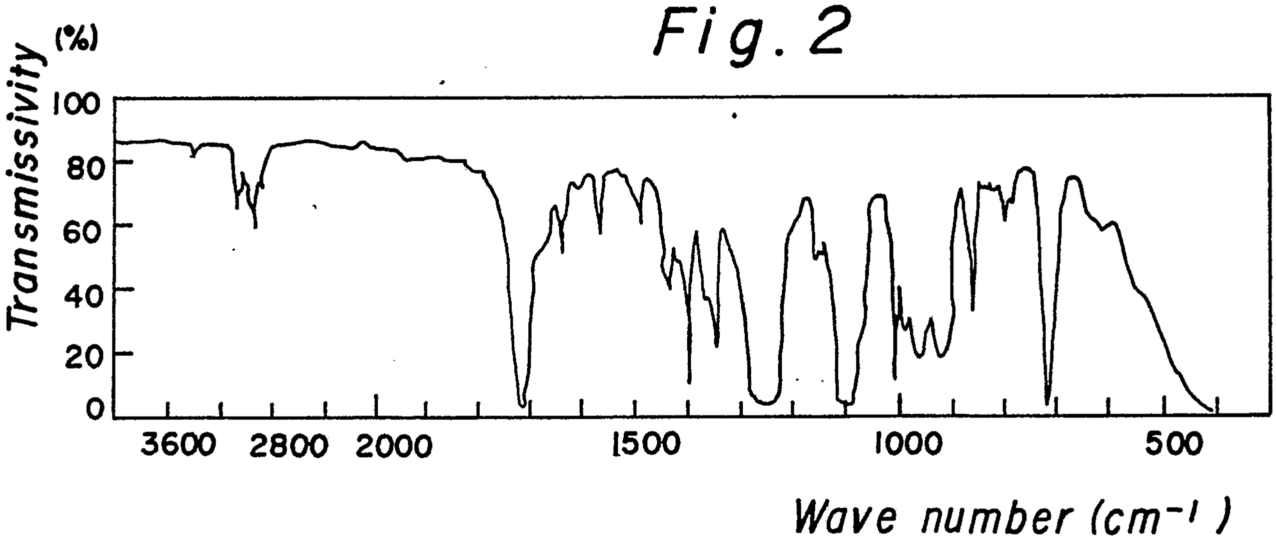
Transmissivity (%)
100
80
60
40
20
0
3600
2800
2000
1500
1000
500
Wave number ($cm^{-1}$)

Fig. 3

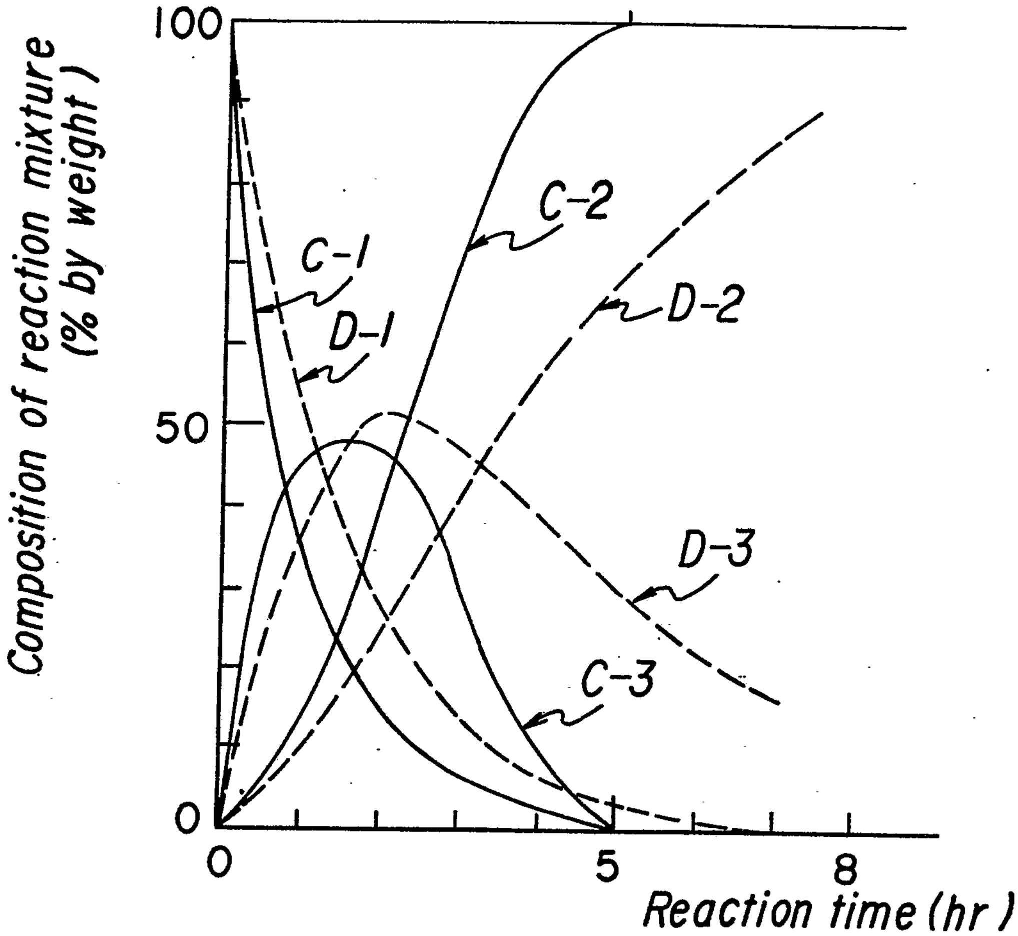
100
50
0
Composition of reaction mixture (% by weight)
C-1
D-1
C-2
D-2
D-3
C-3
0
5
8
Reaction time (hr)

Fig. 4

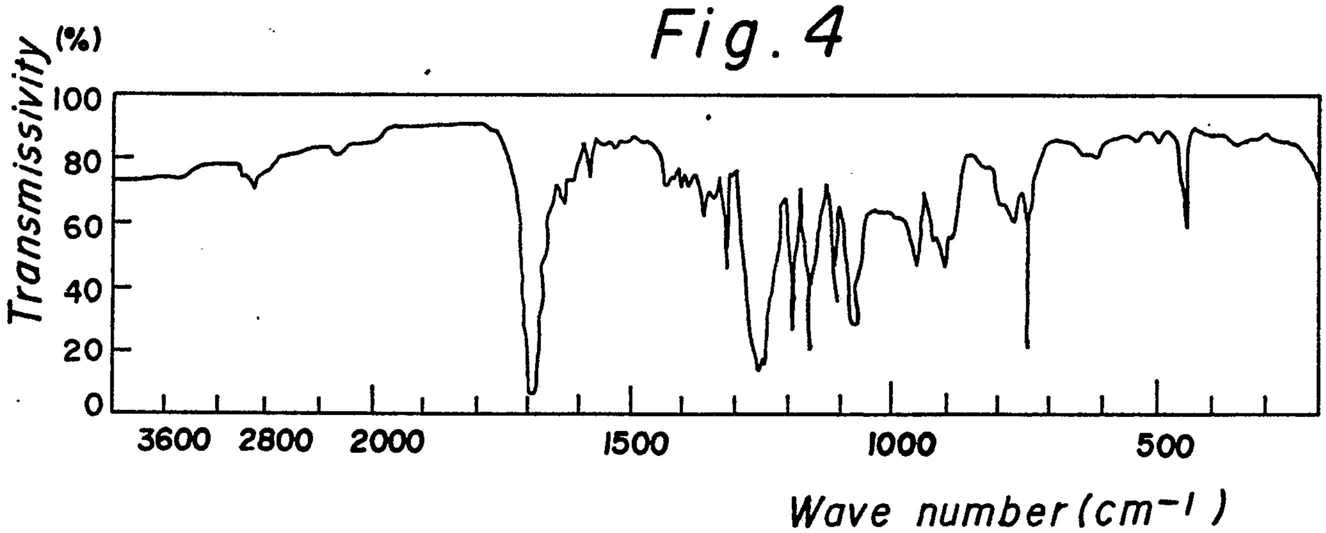
Transmissivity (%)
100
80
60
40
20
0
3600
2800
2000
1500
1000
500
Wave number (cm⁻¹)